# EUROPEAN PATENT SPECIFICATION

(11) **EP 1 991 225 B1**
(45) Date of publication and mention of the grant of the patent: **06.11.2013**
(21) Application number: 07701800.0
(22) Date of filing: 21.02.2007
(51) Int. Cl.: A61K 31/195, A61K 31/4196, A61K 31/4412

(54) **THE USE OF DEFERIPRONE AND METHODS TO TREAT AND/OR PREVENT FRIEDREICH ATAXIA RESULTING FROM INTRACELLULAR MISHANDLING OF IRON**
VERWENDUNG VON DEFERIPRON UND VERFAHREN ZUR BEHANDLUNG UND/ODER PRÄVENTION VON FRIEDREICH-ATAXIE DURCH INTRAZELLULÄRE FEHLBEHANDLUNG VON EISEN
UTILISATION DE DÉFÉRIPRONE ET MÉTHODES DE TRAITEMENT PROPHYLACTIQUE ET/OU THÉRAPEUTIQUE DE LA MALADIE DE FRIEDREICH RÉSULTANT D'UN DYSFONCTIONNEMENT DU TRAITEMENT INTRACELLULAIRE DU FER

(30) Priority: 22.02.2006 US 775320 P
(43) Date of publication of application: 19.11.2008
(73) Proprietor: Apotex Technologies Inc., Toronto, ON M9L 1T9 (CA)
(72) Inventor: Munnich, Arnold, 75743 Paris Cedex 15 (FR); Spino, Michael, Toronto, ON M9L 2Z7 (CA); Cabantchik, Ioav, Givat Ram Jerusalem 91904 (IL)
(74) Representative: Howard, Paul Nicholas
(86) International application number: PCT/CA2007/000252
(87) International publication number: WO 2007/095728

(56) References cited:
- WO-A-02/02114
- US-A1- 2006 030 619
- US-A1- 2006 234 927
- KONTOGHIORGHES G J ET AL: "THE DESIGN AND DEVELOPMENT OF DEFERIPRONE (L1) AND OTHER IRON CHELATORS FOR CLINICAL USE: TARGETING METHODS AND APPLICATION PROSPECTS" CURRENT MEDICINAL CHEMISTRY, BENTHAM SCIENCE PUBLISHERS BV, BE, vol. 11, no. 16, 1 August 2004 (2004-08-01), pages 2161-2183, XP009042933 ISSN: 0929-8673
- LOVEJOY DAVID B ET AL: "PCTH: A novel orally active chelator for the treatment of iron overload disease" HEMOGLOBIN,, vol. 30, no. 1, 1 February 2006 (2006-02-01), pages 93-104, XP009118739 ISSN: 0363-0269
- RICHARDSON: 'The Therapeutic Potential of Iron Chelators' EXPERT OPINION ON INVESTIGATIONAL DRUGS vol. 8, no. 12, 1999, pages 2141 - 2158, XP008102673
- RICHARDSON: 'Friedreich's Ataxia: Iron Chelators that Target the Mitochndrion as a Therapeutic Strategy?' EXPERT OPINION ON INVESTIGATIONAL DRUGS vol. 12, no. 2, 2003, pages 235 - 245, XP008102655

## Description

### FIELD OF INVENTION

The present invention relates to a method of treating or preventing disorders associated with cellular mishandling of iron and more particularly neuro-degenerative diseases such as Friedreich Ataxia in the absence of generalized iron overload. More particularly, the invention relates to the administration of iron chelators currently used for the treatment of iron overload, which have now been shown and established to safely remove excess iron from the mitochondria of cells to minimize intracellular and intra-mitochondrial iron-induced cellular and subcellular damage, including relevant iron chelators deferiprone and deferasirox.

### BACKGROUND OF THE INVENTION

Friedreich ataxia is a degenerative disease with autosomal recessive inheritance, where the cardinal features include progressive limb and gait ataxia, areflexia and pyramidal signs in the legs and hypertrophic cardiomyopathy. It was first described in 1863 by Nikolaus Friedreich, a professor of medicine in Heidelberg, Germany, when he presented six patients in two families (Friedreich N. Ueber degenerative atrophie der spinalen Hinterstra¨nge. Virchow's Arch path Anat 1863;26:391-419, 433-59, and 1863;27:1-26 in Pearce JM. Friedreich's ataxia. J Neurol Neurosurg Psychiatry. 2004 May;75:688). The incidence is reported to be about 1 per 30,000 live births, primarily in Caucasian populations (Delatycki et al. Friedreich ataxia: an overview. J Med Genet 2000; 37: 1-8; and Delatycki et al. Friedreich ataxia: from genes to therapies? Most cases are caused by a single mutation, paving the way for therapeutic advances for this fatal disease. Med. J.Aust. 2005, Vol. 182 (9):439).

Friedreich ataxia results from a mutation of a gene locus on chromosome 9q13 (Chamberlain et al et al. Mapping of mutation causing Friedreich's ataxia to human chromosome 9. Nature 1988; 334: 248-50). The frataxin gene codes for a 210 amino acid protein of unknown function; the mutation is an unstable expansion of a GAA repeat in the first intron inherited from both parents (Campuzano et al. Friedreich's ataxia: autosomal recessive disease caused by an intronic GAA triplet repeat expansion. Science 1996;271:1423-7). Friedreich ataxia results from a deficiency, but not total absence of frataxin in cells of the brain, nerves, heart, and pancreas (Becker and Richardson. Frataxin: its role in iron metabolism and the pathogenesis of Friedreich's ataxia. Int J Biochem Cell Biol. 2001 Jan;33:1-10). Death occurs about 36 years after the onset of the disease and is due primarily to hypertrophic cardiomyopathy (Voncken et al. Friedreich ataxia-update on pathogenesis and possible therapies. Neurogenetics 2004; 5: 1-8).

Histopathological and magnetic resonance imaging (MRI) studies have shown that iron accumulates in heart muscle, spinocerebellar tracts (dentate nuclei) and spinal cord of patients with Friedreich ataxia. When there is insufficient frataxin, there is a deficit in iron-sulphur protein clusters resulting in an accumulation of labile iron leading to oxidative damage in spinocerebellar tracts, spinal cord and heart muscle (Rotig et al. Aconitase and mitochondrial iron-sulphur protein deficiency in Friedreich ataxia. Nat Genet 1997;17:215-7; Delatycki et al.; Direct evidence that mitochondrial iron accumulation occurs in Friedreich ataxia. Ann Neurol 1999;45:673-5). Idebenone, a short-chain quinone analogue, acting as a potent free-radical scavenger, protects heart muscle from free radical-induced injury, but has failed to improve or even stabilize ataxia and other neurological symptoms. (Rustin et al. Effect of idebenone on cardiomyopathy in Friedreich's ataxia: a preliminary study. Lancet 1999;354:477-9).

In conditions of iron overload, such as transfusion-dependent patients with thalassemia, iron accumulates throughout the body and damage to liver, heart and endocrine organs become apparent in the second or third decade of life. Thus patients are administered iron chelators to reduce the total body iron load, and, where possible have an effect on specific organs, such as the liver or heart (Rund and Rachmilewitz. β-thalassemia. New Engl J Med 2005;353:1135-46). Patients are monitored to assess the level of iron in the body by periodic serum ferritin concentration measurements and hepatic iron concentration determinations following biopsy, MRI or SQUID assessments. Serum ferritin concentrations are typically well over 1000 mcg/L and liver iron concentrations > 7 mg/g dry weight of liver are typical.

Over the past decade, 2 iron chelators have proved useful in clinical practice and a third has recently been submitted to regulatory agencies around the world for licensure (Hershko et al. Iron Overload and Chelation. Hematology, 2005; 10 Supplement 1: 171-173). Desferrioxamine is a potent iron chelator that is not adequately absorbed following oral administration and thus must be administered parenterally. It lowers total body iron as assessed by a decline in both the serum ferritin concentration and hepatic iron concentration, and in heart iron as well. Deferiprone is an orally absorbed iron chelator and has also been shown to reduce total body iron as assessed by the same indices. In addition, it has a preferential effect on reduction of heart iron. A third iron chelator, deferasirox, also absorbed orally, is demonstrating clinical benefits in conditions of iron overload and has recently been approved for licensure in the US.

This picture is in contrast to Friedreich ataxia, where the total body iron load is not measurably increased. Serum ferritin concentrations are typically <100 mcg/L and liver iron concentrations are "normal". Thus while patients may experience mitochondrial iron-induced cellular damage, there is no generalized tissue iron overload. Since iron is a critical component of many biochemical processes, there is a concern that the administration of an iron chelator in conditions of non-iron overload, may lead to serious toxicity. Indeed, the "Porter index" (Porter. A risk-benefit assessment of iron-chelation therapy. Drug Saf. 1997;17:407-21) was proposed as a guide to minimize desferrioxamine toxicity in transfused thalassemia patients by indexing the dose of desferrioxamine to the serum concentration of ferritin as a measure of the degree of iron overload. A decreased dose, or even interruption in the use of desferrioxamine is proposed when there is a decline in the serum ferritin concentration. Often, a decline in serum ferritin below 500 mcg/L leads to a cessation in chelation therapy. Such patients still remain iron-overloaded compared to normal patients, or even those with Friedreich ataxia, but the level of loading is not sufficiently increased to justify the risk of iron chelation therapy. Such a concern is the rationale upon which some have challenged the concept of using iron chelators for Friedreich ataxia or other conditions of cellular iron mishandling, in the absence of generalized iron overload (Wilson et al. Normal serum iron and ferritin concentrations in patients with Friedreich's ataxia. Ann Neurol. 1998; 44:132-4.).

Initially, it seemed that iron played a critical role in the development of Friedreich ataxia symptomatology, and that its removal from the mitochondrion might alleviate the condition. However, with increasing knowledge about the disease and the relevant molecular biology, this view was rejected and significant doubt was cast that an iron chelator would provide a net beneficial result, because of the lack of evidence of a preferential effect on the mitochondrion (Delatycki et al. Friedreich ataxia: from genes to therapies? Med J Australia. 2005; 182:439); Sturm et. al. Friedrieich's Ataxia, No change in Mitochondrial Labile Iron to Human Lymphoblasts and Fibroblasts J Biol Chem. 2005;280:6701-8). That is, if iron were removed, not only from the mitochondrion, but also from the cytosol, interference with critical elements of intermediary metabolism, secondary to a functional loss of iron would be expected, rendering the treatment toxic, instead of beneficial.

Indeed, evidence had already been presented that an iron chelator, desferrioxamine, was actually harmful for the respiratory chain, as it displaced iron from membranes and enhanced the oxidative stress and respiratory chain injury *in vitro* (Rustin et al. Effect of idebenone on cardiomyopathy in Friedreich's ataxia: a preliminary study. Lancet. 1999; 7;354:477-9). It was predictable that an agent that would remove iron from the body, might actually induce toxicity in patients without generalized iron overload.

Furthermore, as more information became available about the genetics of the disease, animal models of Friedreich ataxia were engineered by introducing a deletion of the gene for frataxin, it became apparent to scientists working in the field that iron loading of the mitochondrion may not be a pivotal event. For example, while frataxin deficiency had been identified as a key feature in Friedreich ataxia, and while it was known to store iron in the mitochondria, animal knockout models for frataxin do not accumulate iron in the brain until late in the animal's life, thus relegating iron's role to a secondary one (Chantrel-Groussard et al. Disabled early recruitment of antioxidant defenses in Friedreich's ataxia. Hum Mol Genet. 2001;10:2061-7; Simon et al. Friedreich ataxia mouse models with progressive cerebellar and sensory ataxia reveal autophagic neurodegeneration in dorsal root ganglia. J Neurosci. 2004;24:1987-95).

Thus, with biochemical results of an iron chelator suggesting toxicity and genetic data, including animal knockout models for frataxin, suggesting that iron chelators would not be beneficial, attention in the field turned away from iron chelators as likely therapeutic agents and towards gene therapy (Voncken et al. Friedreich ataxia - update on pathogenesis and possible therapies. Neurogenetics. 2004; 5:1-8).

Notwithstanding the lack of generalized iron overload in Friedreich ataxia, it appears that the intracellular damage in this disease is clearly iron-induced. Within the mitochondrion of a cell, a deficiency in frataxin results in an accumulation of labile iron, leading to oxidative damage. One approach to address this matter has been the use of a cell permeant anti-oxidant, idebenone, which has been reported to reduce some of the symptoms of Friedreich ataxia, particularly those related to heart damage as a result of preventing redox cycling induced by iron (Hausse. Idebenone and reduced cardiac hypertrophy in Friedreich's ataxia. Heart. 2002; 87:346-9). However, no measurable effects on ataxia or other adverse effects of the disease associated with central nervous system abnormalities have been detected with the use of idebenone, thus failing to address ataxia, one of the major debilitating effects of the disease, and the reason most patients become confined to wheelchairs.

Recent studies by Glickstein et al. (Blood 2005) and others reviewed by C. Hershko, G. Link, A. M. Konijn and Z I. Cabantchik Objectives and Mechanism of Iron Chelation Therapy Ann. N.Y. Acad. Sci. 1054: 124-135 (2005) indicated that some permeant iron chelators reduce the production of reactive oxygen species in living cells concomitant with the reduction of their labile iron pool. Previous studies also indicated that at relatively low concentrations chelators with suitable characteristics, such as deferiprone (Breuer et al. Blood. 2001; 97:792-8; Pootrakul et al. Blood. 2004; 104: 1504-10) and deferasirox (Hershko et al. Blood. 2001. 97:1115-22) can mobilize labile iron from cells and tissues and safely transfer it to' physiological acceptors such as transferrin. Such "closed" redistribution of iron was conceived as advantageous for minimizing loss of essential iron by chelators. Moreover, an analogous mechanism is envisaged to be operative in cells, whereby deferiprone could shuttle iron between cell components and bypass steps based on frataxin, including the supply of iron for Fe-S-cluster formation.

Deferiprone (3-hydroxy-1,2-dimethylpyridin-4-one), presently used for the treatment of transfusional iron overload, can cross cell membranes (including the blood brain barrier), gain access to cell organelles including mitochondria, and reduce iron-dependent free radical formation (Glickstein et al. Intracellular labile iron pools as direct targets of iron chelators: a fluorescence study of chelator action in living cells. Blood 2005;106:3242-50). Deferiprone also removes cardiac iron, as measured by MRI T2* and increases left ventricular ejection fraction in transfused thalassemia patients with cardiac iron overload (Pennell et al. Randomized Controlled Trial of Deferiprone or Deferoxamine in Beta-Thalassemia Major Patients with Asymptomatic Myocardial Siderosis. Blood First Edition Paper, prepublished online December 13, 2005; DOI 10.1182/blood-2005-07-2948), suggesting it may also generate cardiac benefits in patients with iron-induced cardiac damage.

Kontoghiorghes, G.J., et al., Curr. Med. Chem., 11, 2004, 2161-2183 discloses deferiprone and other iron chelators for the treatment of iron overloading conditions and non iron loading conditions affected by iron containing enzymes.

US 2006/0030619 discloses deferiprone and other iron chelators for the treatment of a number of disorders, including neurodegenerative disorders such as Parkinson's disease and Friedreich's ataxia.

### List of references cited

1. Pearce JM. Friedreich's ataxia. J Neurol Neurosurg Psychiatry. 2004;75:688.
2. Delatycki MB, Williamson R, Forrest SM. Friedreich ataxia: an overview. J Med Genet. 2000; 37:1-8.
3. Delatycki MB, Ioannou,P.A.; Churchyard,A.J. Friedreich ataxia: from genes to therapies? Most cases are caused by a single mutation, paving the way for therapeutic advances for this fatal disease. Med. J.Aust. 2005, Vol. 182 (9):439.
4. Chamberlain S, Shaw J, Rowland A, Wallis J, South S, Nakamura Y, von Gabain A, Farrall M, Williamson R. Mapping of mutation causing Friedreich's ataxia to human chromosome 9. Nature. 1988; 334:248-50.
5. Voncken,M.; Ioannou,P.; Delatycki,M.B. Friedreich ataxia-update on pathogenesis and possible therapies. Neurogenetics.2004; 5:1-8.
6. Becker E, Richardson DR. Frataxin: its role in iron metabolism and the pathogenesis of Friedreich's ataxia. Int J Biochem Cell Biol. 2001 Jan;33:1-10.
7. Waldvogel D, van Gelderen P, Hallett M. Increased iron in the dentate nucleus of patients with Friedrich's ataxia. Ann Neurol 1999; 46:123-5.
8. Campuzano V, Montermini L, Molto MD, et al. Friedreich's ataxia: autosomal recessive disease caused by an intronic GAA triplet repeat expansion. Science 1996; 271:1423-7.
9. Rotig A, de Lonlay P, Chretien D, et al. Aconitase and mitochondrial iron-sulphur protein deficiency in Friedreich ataxia. Nat Genet 1997; 17:215-7.
10. Delatycki MB, Camakaris J, Brooks H, et al. Direct evidence that mitochondrial iron accumulation occurs in Friedreich ataxia. Ann Neurol 1999; 45:673-5.
11. Rustin P, von Kleist-Retzow JC, Chantrel-Groussard K, Sidi D, Munnich A, Rotig A. Effect of idebenone on cardiomyopathy in Friedreich's ataxia: a preliminary study. Lancet 1999; 354:477-9.
12. Rund,D.; Rachmilewitz,E. Beta-thalassemia. N.Engl.J.Med. 2005; 353:1135- 1146
13. Hershko,C.; Link,G.; Konijn,A.M.; Ioav,Cabantchik Z. Iron overload and chelation. Hematology. 2005; 10 Suppl 1: 171-173.
14. Porter,J.B. A risk-benefit assessment of iron-chelation therapy. Drug Safety 1997; 17:407-21
15. Chantrel-Groussard K, Geromel V, Puccio H, Koenig M, Munnich A, Rotig A, Rustin P. Disabled early recruitment of antioxidant defenses in Friedreich's ataxia. Hum Mol Genet. 2001; 10:2061-7.
16. Hausse AO, Aggoun Y, Bonnet D, Sidi D, Munnich A, Rotig A, Rustin P. Idebenone and reduced cardiac hypertrophy in Friedreich's ataxia. Heart. 2002; 87:346-9.
17. Franchini M, Veneri D. Iron-chelation therapy: an update. Hematol J 2004;5:287-92.
18. Glickstein H, El RB, Shvartsman M, Cabantchik ZI. Intracellular labile iron pools as direct targets of iron chelators: a fluorescence study of chelator action in living cells. Blood 2005;106:3242-50.
19. Pennell DJ, Berdoukas V, Karagiorga,M et al.. Randomized controlled trial of deferiprone or deferoxamine in beta-thalassemia major patients with asymptomatic myocardial siderosis. Blood 2005 Dec 13; [Epub ahead of print]
20. Cano SJ, Hobart JC, Hart PE, Korlipara LV, Schapira AH, Cooper JM. International cooperative ataxia rating scale (ICARS): Appropriate for studies of Friedreich's ataxia? Mov Disord 2005;20:1585-91.
21. Richardson DR. Friedreich's ataxia: iron chelators that target the mitochondrion as a therapeutic strategy Expert Opin Investig Drugs. 2003 Feb;12:235-45.
22. Richardson DR, Mouralian C., Ponka P, Becker E. Development of potential iron chelators for the treatment of Friedreich's ataxia: ligands that mobilize mitochondrial iron. Biochimica et Biophysica Acta 2001;1536;133-140.
23. Richardson DR. Novel chelators for central nervous system disorders that involve alterations in the metabolism of iron and other metal ions. Ann N Y Acad Sci. 2004;1012:326-41.
24. Haacke EM, Cheng NY, House MJ, et al. Imaging iron stores in the brain using magnetic resonance imaging. Magn Reson Imaging 2005;23:1-25.
25. Simon D, Seznec H, Gansmuller A, et al. Friedreich ataxia mouse models with progressive cerebellar and sensory ataxia reveal autophagic neurodegeneration in dorsal root ganglia. J Neurosci 2004;24:1987-95.
26. Sturm B, Bistrich U, Schranzhofer M et. al. Friedreich's Ataxia, No Changes in Mitochondrial Labile Iron in Human Lymphoblasts and Fibroblasts. Journal of Biological Chemistry, 2005; 280: 6701-08.
27. Hershko C, Link G, Konjin A and Cabantchik I. Objectives and Mechanism of Iron Chelation Therapy. Ann NY Acad Sci. 2005; 1054: 124-35.
28. Breuer W, Ermers MJ, Pootrakul P, et al. Desferrioxamine-chelatable iron, a component of serum non-transferrin-bound iron, used for assessing chelation therapy. Blood, 2001; 97: 792-8.
29. Pootrakul P, Breuer W, Sametband M, et al. Labile plasma iron (LPI) as an indicator of chelatable plasma redox activity in iron-overloaded -thalassemia/HbE patients treated with an oral chelator. Blood. 2004; 104: 1504-10.
30. Hershko C. ICL670A: a new synthetic oral chelator: evaluation in hypertransfused rats with selective radioiron probes of hepatocellular and reticuloendothelial iron stores and in iron-loaded rat heart cells in culture. Blood. 2001; 97: 1115-22.
31. Gakh O, Park S, Liu G, Macomber L, Imlay JA, Ferreira GC, Isaya G. Mitochondrial iron detoxification is a primary function of frataxin that limits oxidative damage and preserves cell longevity. Human Molecular Genetics 2006 15(3):467-479; doi: 10.1093/hmg/ddi461 (published on the web on December 21, 2005).
32. Wilson RB, Lynch DR, Fischbeck KH. Normal serum iron and ferritin concentrations in patients with Friedreich's ataxia. Ann Neurol. 1998; 44:132-4.
33. Richardson D, Bernhardt PV and Becker EM. University of Queensland, The Heart Research Institute Ltd., US Patent No. 6,989,397 B1, January 24, 2006.
34. Kontoghiorghes G.J., Pattichis K, Neocleous K, and Kolnagou A. The Design and Development of Deferiprone (L1) and Other Iron Chelators for Clincial Use: Targeting Methods and Application Prospects, Current Medicinal Chemistry, 2004.
35. Lodi, R, et. Antioxidant Treatment Improves In Vivo Cardiac and Skeletal Muscle Bioenergetics in Patients with Friedreich's Ataxia, Wiley-Liss, Inc., 2001.

### List of Patents cited:

1. United States Patent No. 6,956,028, "Compositions and Methods for Treatment of Mitochondrial Diseases" Wellsat Therapeutics Corporation, October 18, 2005.
2. United States Patent No. 6,472,378, "Compositions and Methods for Treatment of Mitochondrial Diseases" Pro-Neuro, Inc., October 29, 2002.
3. United States Patent No. 6,133,322, "Quinone Derivatives for Treating or Preventing Diseases Associated with Iron Overload", October 17, 2000.
4. United States Patent No. 5,928,885, "Kit for Detecting Alzheimer's Disease" The McLean Hospital Corporation, July 27, 1999.

Referring now to United States Patent 6,989,397 granted January 24, 2006 to University of Queensland, invented by Des Richardson et al., there is taught 2-pyridylcarboxaldehyde isonicotinoyl hydrazone (PCIH) analogues which may be suitable if safe for use *in vivo* as iron chelators, for the treatment of iron overload diseases. Both thalassemia and Friedreich Ataxia are identified as diseases for which these iron chelators may be used, although no such compounds are yet available for clinical use in these conditions. Specifically the alleged advantages of the PCIH analogues are discussed in comparison to currently available iron chelators, desferrioxamine and deferiprone.

The patent describes the deficiencies in desferrioxamine at column 2 line 38 onward. Further the patent describes the deficiencies with deferiprone at the same location as follows:
*"The need for an orally effective and economical Fe chelator has recently been emphasized by the failure of deferiprone (also known as L1 or 1,2-dimethyl-3-hydroxyprid-4-one) to successfully chelate Fe from Fe-overload patients (*Olivieri et al., 1988*, New Eng.J.Med. 337 417-23*). In fact, treatment of patients with this later drug resulted in hepatic fibrosis and an increase in liver Fe levels."* * *correct date is 1998.*

Clearly therefore the teaching of Richardson et al. point away from the use of deferiprone as an iron chelator for treatment of patients. We have however discovered this conclusion to be false for the reasons that follow in this specification.

Nowhere in the literature is it taught that it is the function of chelating agents, such as deferiprone or deferasirox, capable of reducing labile mitochondrial iron stores, to benefit patients with Friedreich ataxia by reducing mitochondrial iron-induced intracellular damage, when administered to patients having said disease, and particularly without inducing generalized toxicity.

On the basis of prior observations previously set out herein we postulated that certain suitable iron chelators, having the appropriate characteristics to enable them to cross cell membranes and scavenge organellar labile iron, might reduce mitochondrial iron-induced cellular damage *in vivo* in Friedreich ataxia and protect the central nervous system and the heart from oxidative injury without affecting the iron status of other organs.

It is therefore an object of this invention to use chelating agents capable of reducing labile mitochondrial iron stores, such as for example deferiprone or deferasirox or a physiologically acceptable salt thereof, for treating and/or preventing iron-induced intracellular damage in a patient.

It is a further object of the invention to provide a method of treating, reducing, reversing and or preventing iron-induced neuro-degenerative disease in a patient.

Further and other objects of the invention will become apparent to those skilled in the art when considering the following summary of the invention and the more detailed description of the embodiments of the invention described herein.

### SUMMARY OF THE INVENTION

The current thinking of the leaders in the field is that iron chelators would not be helpful (as iron accumulation was not considered the critical factor in the disease), and might even be harmful to patients with no overt iron overload (as no chelator or chelation regimen was conceived or proposed by them as selective for relieving the relevant cells affected specifically from accumulated iron due to frataxin deficiency). We felt that they may have misjudged the situation, and the potential gain for the patients would be so great that we should conduct preliminary studies to determine if our understanding of the potential benefit of specific chelation therapy, based on agents such as deferiprone, could improve the fundamental problem and/or symptoms of Friedreich ataxia.

In support of our concept of using cell-permeant iron chelators, such as deferiprone or deferasirox, to address the mitochondrial deficiency in Friedreich ataxia as a primary defect, we refer to a new study reporting on the probable role of frataxin in detoxifying iron in the mitochondria as proposed by Gakh et al. (Mitochondrial iron detoxification is a primary function of frataxin that limits oxidative damage and preserves cell longevity. Human Molecular Genetics 2006 15(3):467-479; doi: 10.1093/hmg/ddi461 (published on the web on December 21, 2005). While dealing only *with in vitro* systems, their paper supports the concepts related to one of the mechanisms of action seen in our novel study of the use of deferiprone in the treatment of Friedreich ataxia as described below.

According to a primary aspect of the inventions there is provided deferiprone or physiologically acceptable salts thereof for use as defined in claim 1.

According to another aspect of the invention there is provided a therapeutically effective amount of deferiprone or physiologically acceptable salts thereof to preferentially reduce or render inactive the toxic iron stores in the brain and/or to mitigate the cellular or intracellular mishandling of iron in the brain for the prevention of iron-induced damage.

According to another aspect of the invention there is provided a therapeutically effective amount of deferiprone or physiologically acceptable salts thereof to preferentially reduce or render inactive the toxic iron stores in the brain and/or to mitigate the cellular or intracellular mishandling of iron in the brain for the stabilization of iron-induced damage.

According to another aspect of the invention there is provided a therapeutically effective amount of deferiprone or physiologically acceptable salts thereof to preferentially reduce or render inactive the toxic iron stores in the brain and/or to mitigate the cellular or intracellular mishandling of iron in the brain for the treatment of iron-induced damage.

According to another aspect of the invention there is provided a therapeutically effective amount of deferiprone or physiologically acceptable salts thereof to preferentially reduce or render inactive the toxic iron stores in the brain and/or to mitigate the cellular or intracellular mishandling of iron in the brain for the reversal of iron-induced damage.

In one embodiment the condition being treated is Friedreich ataxia.

According to another aspect of the invention there is provided a therapeutically effective amount of deferiprone or physiologically acceptable salts thereof to preferentially reduce or render inactive the toxic iron stores in the mitochondria for the prevention of mitochondrial iron-induced damage.

According to another aspect of the invention there is provided a therapeutically effective amount of deferiprone or physiologically acceptable salts thereof to preferentially reduce or render inactive the toxic iron stores in the mitochondria for the stabilization of mitochondrial iron-induced damage.

According to another aspect of the invention there is provided a therapeutically effective amount of deferiprone or physiologically acceptable salts thereof to preferentially reduce or render inactive the toxic iron stores in the mitochondria for the treatment of mitochondrial iron-induced damage.

According to another aspect of the invention there is provided a therapeutically effective amount of deferiprone or physiologically acceptable salts thereof to preferentially reduce or render inactive the toxic iron stores in the mitochondria for the reversal of mitochondrial iron-induced damage.

According to another aspect of the invention there is provided the use of deferiprone or physiologically acceptable salts thereof in the manufacture of a pharmaceutical for prevention of symptoms of Friedreich ataxia.

According to another aspect of the invention there is provided the use of deferiprone or physiologically acceptable salts thereof in the manufacture of a pharmaceutical for stabilization of symptoms of Friedreich ataxia.

According to another aspect of the invention there is provided the use of deferiprone or physiologically acceptable salts thereof in the manufacture of a pharmaceutical for reduction of symptoms of Friedreich ataxia.

According to another aspect of the invention there is provided the use of deferiprone or physiologically acceptable salts thereof in the manufacture of a pharmaceutical for treatment of Friedreich ataxia.

Disclosed herein is a method of treating Friedreich ataxia in patients resulting from mitochondrial iron-induced damage, comprising administering to the patient a therapeutically effective amount of deferiprone or physiologically acceptable salts thereof sufficient to treat Friedreich ataxia resulting from mitochondrial iron-induced damage.

Disclosed herein is a method of preventing the development of symptoms of Friedreich ataxia in patients resulting from mitochondrial iron-induced damage, comprising administering to the patient a therapeutically effective amount of deferiprone, physiologically acceptable salts thereof sufficient to prevent symptoms Friedreich ataxia.

Disclosed herein is a method of reducing symptoms of Friedreich ataxia in patients resulting from mitochondrial iron-induced damage, comprising administering to the patient a therapeutically effective amount of deferiprone or physiologically acceptable salts thereof sufficient to reduce symptoms of Friedreich ataxia.

Disclosed herein is a method of stabilizing the symptoms of Friedreich ataxia in patients resulting from mitochondrial iron-induced damage, comprising administering to the patient a therapeutically effective amount of deferiprone or physiologically acceptable salts thereof sufficient to stabilize the symptoms of Friedreich ataxia.

According to another aspect of the invention there is provided for use to treat Friedreich ataxia in a patient resulting from mitochondrial iron-induced damage, comprising administering to the patient a therapeutically effective amount of deferiprone or physiologically acceptable salts thereof sufficient to treat Friedreich ataxia.

According to yet another aspect of the invention there is provided for use to prevent the development of symptoms of Friedreich ataxia in a patient resulting from mitochondrial iron-induced damage, comprising administering to the patient a therapeutically effective amount of deferiprone or physiologically acceptable salts thereof sufficient to prevent the development of symptoms of Friedreich ataxia.

According to yet another aspect of the invention there is provided for use to stabilize symptoms of Friedreich ataxia in a patient resulting from mitochondrial iron-induced damage, comprising administering to the patient a therapeutically effective amount of deferiprone or physiologically acceptable salts thereof sufficient to stabilize symptoms of Friedreich ataxia.

According to yet another aspect of the invention there is provided for use to reduce the symptoms of Friedreich ataxia in a patient resulting from mitochondrial iron-induced damage, comprising administering to the patient a therapeutically effective amount of deferiprone or physiologically acceptable salts thereof sufficient to reduce the symptoms of Friedreich ataxia.

According to another aspect of the invention, there is provided the use of deferiprone or physiologically acceptable salts thereof for the prevention of the development of symptoms of Friedreich ataxia in patients resulting from mitochondrial iron-induced damage.

According to yet another aspect of the invention, there is provided the use of deferiprone or physiologically acceptable salts thereof for the stabilization of symptoms of Friedreich ataxia in patients resulting from mitochondrial iron-induced damage.

According to yet another aspect of the invention, there is provided the use of deferiprone or physiologically acceptable salts thereof for the reduction of symptoms of Friedreich ataxia in patients resulting from mitochondrial iron-induced damage.

According to yet another aspect of the invention, there is provided the use of deferiprone or physiologically acceptable salts thereof for the treatment Friedreich ataxia in patients resulting from mitochondrial iron-induced damage.

According to another aspect of the invention, there is provided an effective therapeutic amount of deferiprone or a physiologically acceptable salt thereof for the prevention of the development of symptoms in Friedreich ataxia in patients resulting from mitochondrial iron-induced damage, sufficient to treat Friedreich ataxia.

According to yet another aspect of the invention, there is provided an effective therapeutic amount of deferiprone or a physiologically acceptable salt thereof for the stabilization of symptoms of Friedreich ataxia in patients resulting from mitochondrial iron-induced damage, sufficient to treat Friedreich ataxia.

According to yet another aspect of the invention, there is provided an effective therapeutic amount of deferiprone or a physiologically acceptable salt thereof for the reduction of symptoms of Friedreich ataxia in patients resulting from mitochondrial iron-induced damage, sufficient to treat Friedreich ataxia.

According to yet another aspect of the invention, there is provided an effective therapeutic amount of deferiprone or a physiologically acceptable salt thereof for the treatment of Friedreich ataxia in patients resulting from mitochondrial iron-induced damage, sufficient to treat Friedreich ataxia.

Disclosed herein is a method of prevention of symptoms of Friedreich ataxia in patients resulting from mitochondrial iron-induced damage, comprising the administration of a therapeutically effective amount of deferiprone or a physiologically acceptable salt thereof sufficient to prevent the symptoms of Friedreich ataxia.

Disclosed herein is a method of stabilization of symptoms of Friedreich ataxia in patients resulting from mitochondrial iron-induced damage, comprising the administration of a therapeutically effective amount of deferiprone or a physiologically acceptable salt thereof sufficient to stabilize the symptoms of Friedreich ataxia.

Disclosed herein is a method of reduction of the symptoms of Friedreich ataxia in patients resulting from mitochondrial iron-induced damage, comprising the administration of a therapeutically effective amount of deferiprone or a physiologically acceptable salt thereof sufficient to reduce the symptoms of Friedreich ataxia.

Disclosed herein is a method of treatment of Friedreich ataxia in patients resulting from mitochondrial iron-induced damage, comprising the administration of a therapeutically effective amount of deferiprone or a physiologically acceptable salt thereof sufficient to treat Friedreich ataxia.

According to another aspect of the invention there is provided the use of deferiprone in the manufacture of a pharmaceutical for prevention of symptoms of Friedreich ataxia in patients resulting from mitochondrial iron-induced damage, comprising the administration of a therapeutically effective amount of deferiprone or physiologically acceptable salts thereof sufficient to prevent the development of symptoms of Friedreich ataxia.

According to yet another aspect of the invention there is provided the use of deferiprone in the manufacture of a pharmaceutical for stabilization of symptoms of Friedreich ataxia in patients, comprising the administration of a therapeutically effective amount of deferiprone or physiologically acceptable salts thereof sufficient to stabilize symptoms of Friedreich ataxia in patients resulting from mitochondrial iron-induced damage.

According to yet another aspect of the invention there is provided the use of deferiprone in the manufacture of a pharmaceutical for reduction of symptoms of Friedreich ataxia in patients, comprising the administration of a therapeutically effective amount of deferiprone or physiologically acceptable salts thereof sufficient to reduce the symptoms of Friedreich ataxia in patients resulting from mitochondrial iron-induced damage.

According to yet another aspect of the invention there is provided the use of deferiprone in the manufacture of a pharmaceutical for treatment of Friedreich ataxia in patients, comprising the administration of a therapeutically effective amount of deferiprone or physiologically acceptable salts thereof sufficient to treat Friedreich ataxia.

According to another aspect of the invention there is provided the use of deferiprone for the prevention of the development of symptoms of Friedreich ataxia in a patient resulting from mitochondrial iron-induced damage, comprising administering to the patient a therapeutically effective amount of deferiprone, or a physiologically acceptable salt thereof in order to reduce the iron stores in the mitochondria.

According to yet another aspect of the invention there is provided the use of deferiprone for the stabilization of symptoms of Friedreich ataxia in a patient resulting from mitochondrial iron-induced damage, comprising administering to the patient a therapeutically effective amount of deferiprone, or a physiologically acceptable salt thereof in order to reduce the iron stores in the mitochondria.

According to yet another aspect of the invention there is provided the use of deferiprone for the treatment of Friedreich ataxia in a patient resulting from mitochondrial iron-induced damage, comprising administering to the patient a therapeutically effective amount of deferiprone, or a physiologically acceptable salt thereof in order to reduce the iron stores in the mitochondria.

According to yet another aspect of the invention there is provided the use of deferiprone for the reversal of symptoms of Friedreich ataxia in a patient resulting from mitochondrial iron-induced damage, comprising administering to the patient a therapeutically effective amount of deferiprone, or a physiologically acceptable salt thereof in order to reduce the iron stores in the mitochondria.

According to yet another aspect of the invention there is provided a therapeutically effective amount of deferiprone or physiologically acceptable salt thereof for the prevention of iron-induced neuro-degenerative disease in patients resulting from mitochondrial iron-induced damage, comprising an effective amount of deferiprone or a physiologically acceptable salt thereof to preferentially reduce the iron stores in the mitochondria.

According to yet another aspect of the invention there is provided a therapeutically effective amount of deferiprone or physiologically acceptable salt thereof for the stabilization of iron-induced neuro-degenerative disease in patients resulting from mitochondrial iron-induced damage, comprising an effective amount of deferiprone or a physiologically acceptable salt thereof to preferentially reduce the iron stores in the mitochondria.

According to yet another aspect of the invention there is provided a therapeutically effective amount of deferiprone or physiologically acceptable salt thereof for the treatment of iron-induced neuro-degenerative disease in patients resulting from mitochondrial iron-induced damage, comprising an effective amount of deferiprone or a physiologically acceptable salt thereof to preferentially reduce the iron stores in the mitochondria.

According to yet another aspect of the invention there is provided a therapeutically effective amount of deferiprone or physiologically acceptable salt thereof for the reversal of iron-induced neuro-degenerative disease in patients resulting from mitochondrial iron-induced damage, comprising an effective amount of deferiprone or a physiologically acceptable salt thereof to preferentially reduce the iron stores in the mitochondria.

In the disclosed methods the active ingredient is deferiprone or physiologically acceptable salts thereof for preventing the development of symptoms of Friedreich ataxia resulting from mitochondrial iron-induced damage in patients.

In the disclosed methods the active ingredient is deferiprone or physiologically acceptable salts thereof for stabilizing the symptoms of Friedreich ataxia resulting from mitochondrial iron-induced damage in patients.

In the disclosed methods of the invention the active ingredient is deferiprone or physiologically acceptable salts thereof for treating Friedreich ataxia resulting from mitochondrial iron-induced damage in patients.

In the disclosed methods the active ingredient is deferiprone or physiologically acceptable salts thereof for reducing the symptoms of Friedreich ataxia resulting from mitochondrial iron-induced damage in patients.

In another embodiment of the use of the invention the active ingredient is deferiprone or physiologically acceptable salts thereof for preventing the symptoms of Friedreich ataxia resulting from mitochondrial iron-induced damage in patients.

In yet another embodiment of the use of the invention the active ingredient is deferiprone or physiologically acceptable salts thereof for stabilizing the symptoms of Friedreich ataxia resulting from mitochondrial iron-induced damage in patients.

In yet another embodiment of the use of the invention the active ingredient is deferiprone or physiologically acceptable salts thereof for treating Friedreich ataxia resulting from mitochondrial iron-induced damage in patients.

In yet another embodiment of the use of the invention the active ingredient is deferiprone or physiologically acceptable salts thereof for reducing the symptoms of Friedreich ataxia resulting from mitochondrial iron-induced damage in patients.

In another embodiment the deferiprone or physiologically acceptable salts thereof may further comprise an oral dosage form with other excipients.

In yet another embodiment of the invention the use may further comprise an oral dosage form of deferiprone or physiologically acceptable salts thereof with other excipients.

The deferiprone or a physiologically acceptable salt thereof for use as defined in claim 1 comprises daily administration of in the range of 20 mg/kg to 30 mg/kg, optionally daily administration of 20 mg/kg or 30 mg/kg.

Preferably deferiprone is administered in a manner selected from the group of intravenously, transdermally, rectally, orally, bucally, or aurally.

In yet another embodiment of the invention the use may further comprise deferiprone administered in a manner selected from the group of intravenously, transdermally, rectally, orally, bucally, or aurally.

Preferably deferiprone is administered orally.

In one embodiment of the methods or uses of the invention the dosage form is a modified release formulation, including sustained release.

In another embodiment of the methods or uses of the invention deferiprone is administered in addition to other regimens.

### BRIEF DESCRIPTION OF THE FIGURES

Figure 1 is an MRI visualisation of iron accumulation in dentate nuclei of patients with Friedreich ataxia
Figure 2 represents a time course of mean R2* values in the left and right dentate nuclei of patients with Friedreich ataxia receiving deferiprone

### DETAILED DESCRIPTION OF THE INVENTION

Two critical factors remained outstanding and unproven that prevented our use of an iron chelator in general, and deferiprone, in particular, in the treatment of Friedreich ataxia. The first was whether patients without generalized iron overload could safely be administered doses of the drug that could remove iron from various tissues. The second was how effective was deferiprone to specifically remove mitochondrial iron, not just in an *in vitro* situation evaluating a cell system, but *in vivo?* To address both questions, we conducted studies in animals.

### Studies of Iron Chelation in Non-Iron Overloaded Monkeys

As part of a larger study on the potential toxicity of iron chelators, we evaluated the toxicity of deferiprone in normal monkeys receiving 150 mg/kg/day (75 mg/kg twice daily) for 1 year.

### 1.1. Objective

To determine the toxicity of deferiprone in non-iron-loaded cynomolgus monkeys following twice daily oral (gavage) administration for 52 consecutive weeks, and to evaluate the regression of any toxic effect during a 4-week treatment-free period.

### 1.2. Methods.

Groups of 4 male and 4 female monkeys (2 to 3 years old) were dosed by nasogastric intubation at (0.5% w/v aqueous carboxymethylcellulose vehicle) 75 mg/kg deferiprone twice daily (bid), with 6-8 hours between doses, for at least 364 days.

### 1.3. Monitoring.

Clinical signs, body weights and food intake were assessed at frequent intervals. Ophthalmological and cardiovascular examinations were carried out in Weeks 17, 30, 43, 56 and 60. Hematology, coagulation, clinical chemistry and urine parameters were measured at baseline and in Weeks 8, 16, 30, 41, 56 and 60. Blood samples were taken on the first day of dosing, and in Weeks 17 and 56 for assessment of the serum deferiprone time-concentration profile. A full necropsy was performed on each animal, selected organs weighed, and histopathological examination of abnormalities and selected tissues conducted.

### 1.4. Results.

Clinical signs in animals were incidental to administration of deferiprone by the IP route. There were no deferiprone-related effects on body weight, food intake, ophthalmology, cardiac conduction, blood pressure, heart rate, and hematology or urine composition.

Deferiprone was detectable (HPLC) from 0.5 h after dosing, and for up to 7 h. Mean peak concentrations ranged from 25 to 30 mcg/ml throughout the 52 week period of treatment, representing concentrations that were about three times peak concentrations observed in thalassemia patients treated with a standard dose of 25 mg/kg three times daily. Serum half-life ranged from 0.35-2.39 h in individuals.

No macroscopic findings related to deferiprone were found. No microscopic findings in non-iron-loaded monkeys could be attributed to treatment with deferiprone.

### 1.5. Conclusion.

Deferiprone given orally to non-iron-loaded cynomolgus monkeys at 75 mg/kg twice daily for 52 weeks was without significant adverse effect.

The total daily dose used in this study was double the dose normally used to treat iron overloaded patients with thalassemia. These data were unexpected and were critical in establishing the fact that deferiprone could be safely given to primates in the absence of generalized iron overload. Without this information, we could not have recommended that an iron chelator in general, and deferiprone specifically, be attempted in patients with Friedreich ataxia.

### In Vivo Studies of Mitochondrial Iron Removal by Chelation in Rats

As part of a larger study comparing iron chelators, we conducted histologic examination and electron microscopy (EM) of tissues following iron loading (100 mg/kg iron dose intraperitoneally twice weekly for four weeks) in rats (200-250 g) and treatment with deferiprone. Two control groups of rats were studied, one with no iron loading ("Naïve") and the other with iron loading but no iron chelation.

Deferiprone was administered five times weekly (daily Monday to Friday). The animals received 89 doses in 127 days of 100 mg/kg daily, by oral gavage.

Qualitative histological examination of the slides stained with H&E showed no degenerative changes in the liver and heart with iron loading, although there was a large accumulation of iron in the liver, and a random accumulation of iron in the heart.

A large accumulation of electron dense granule-like material was observed in the liver and heart sections of iron-loaded rats. The accumulation of electron-dense material was less apparent in the heart than in the liver. The most prominent iron-loading changes occurred in macrophages (or Kupffer cells) in liver portal tracts. Macrophages in the portal tracts frequently had large accumulation of iron in the matrix of the mitochondria. Mitochondrial membrane structure was irregular with internal structure loss resulting in fusion and enlargement of affected mitochondria. Large amorphous aggregates of degenerate mitochondria with or without vacuolation were seen occasionally in the cytoplasm of severely affected monocytes/macrophages and resembled phagolysosomes. Yet, there was little evidence that the monocytes/macrophages had degenerated with loss of cytoplasmic integrity and dissolution of the cytoplasm and organelles.

The most obvious accumulation in the heart, observed randomly, was seen in the mitochondria of perivascular monocytes (macrophages). The severity of the accumulation was less than that observed in the monocytes of the liver. Thus it was clear that in addition to generalized iron overload, there was excess iron in the mitochondria, the latter condition relevant to Friedreich ataxia.

The administration of deferiprone decreased the iron levels in the liver and heart and most other organs as illustrated in the following table.

Iron content in micrograms per gram dry tissue ± SD as determined by ICP-MS

| | **Heart** | **Liver** | **Kidney** | **Thyroid** | **Lung** | **Skeletal Muscle** |
|---|---|---|---|---|---|---|
| **Naive** | 276±25 | 376±127 | 305±80 | 95±13 | 376±45 | 50±17 |
| **Fe-control** | 1,088±36 | 11,534±554 | 801±39 | 583±275 | 1,234±227 | 205±109 |
| **Deferiprone** | 586±143 | 7,780±1,637 | 642±104 | 425±228 | 1065±101 | 146±49 |

The data demonstrated that iron loading resulted in an accumulation of iron in the mitochondria of these animals and that deferiprone has the ability to reduce iron loading in key organs.

An integral component to the meaningful interpretation of this study in rats, is the recently published work of one of us (Cabantchik), pertaining to intracellular iron removal using fluorescence probes (Glickstein et al. Intracellular labile iron pools as direct targets of iron chelators: a fluorescence study of chelator action in living cells. Blood. 2005;106:3242-50). The study was designed to evaluate the capacity of clinically important iron chelators, such as deferiprone, desferrioxamine, and deferasirox to: (a) gain direct access to intracellular iron pools of key cells of iron accumulation (macrophages, hepatocytes, and cardiomyocyte cell lines); (b) chelate the labile iron present in discrete cell compartments/organelles; and (c) prevent labile iron involvement in the generation of reactive oxidant species.

The study revealed that chelation by desferrioxamine is slow, although enhanced in cells with relatively high endocytic activities (e.g., hepatocytes), while deferasirox and deferiprone readily enter most cells and efficiently reach the major intracellular sites of iron accumulation.

The above study on animal iron loading, combined with these data of Glickstein et al, demonstrate that deferiprone can remove iron, not only from key organs, but also from the mitochondria, indicating that deferiprone and deferasirox, and thus other chelators with suitable properties of iron-binding and membrane permeability, as abovementioned, should be capable of preventing iron-mediated damage in the mitochondria.

These studies show that deferiprone can remove iron from the mitochondria of various cells, *in vivo*, without generalized cellular or tissue toxicity, and since the mitochondrion is the key intracellular organelle involved in Friedreich ataxia, suggest it might not be toxic if it were used in treating Friedreich Ataxia. Combined with the safety data in 1 year-long treated monkeys, these studies provided the key pieces of information required to overcome the obstacles related to the conduct of a study in which an iron chelator in general, and deferiprone specifically, would be administered to patients with Friedreich ataxia. Thus the following study was conducted as part of the invention.

### EFFECT OF DEFERIPRONE ON ATAXIA AND CEREBELLAR IRON ACCUMULATION IN FRIEDREICH ATAXIA: A PILOT STUDY

In this efficacy-toxicity phase I-II open trial, we showed that oral deferiprone reduced iron concentrations which had been accumulating in the dentate nuclei of cerebella of young patients with Friedreich ataxia and improved their neurological condition.

### Patients and Design

Ten adolescents (four males, six females aged 14 to 23 yrs) with a diagnosis of Friedreich ataxia, confirmed by detection of a trinucleotide-repeat expansion in the first intron of the frataxin gene, were studied. Patients received deferiprone (Ferriprox™, Apotex Inc., Toronto, Canada) orally, in two doses for 1-5 months. Three patients were included, sequentially, for a minimum period of two months at each dose. Patient enrollment was staggered by two-weeks to enable monitoring of response. In the absence of toxicity, and with evidence of efficacy in any of the first 3 patients, a second group of 3 patients would be included at the same dose for a total duration of 6 months. If there was an absence of both efficacy and toxicity, the second group of three patients would be administered a higher dose. If toxicity developed, the current dose would be suspended and the trial resumed with the next group of patients at a lower dose. The patients were on idebenone (10 mg/kg/day, in three doses) for at least two years prior to inclusion and were kept on the drug at the same dose for the duration of the trial. MRI examinations were done at inclusion and at 1, 2, 4 and 6 months. The protocol was promoted by Assistance Publique-Hopitaux de Paris and approved by the local ethical committee and registered at the National Health Authority (AFSSAPS) and at the International Protocol Registration System (www.clinicaltrials.gov). A written informed consent was obtained from patients and parents.

The International Cooperative Ataxia Rating Score (ICARS) was used to assess the symptoms of ataxia before and after 1-5 months (Cano et al. International cooperative ataxia rating scale (ICARS): Appropriate for studies of Friedreich's ataxia? Mov Disord 2005;20:1585-91). This scale has four subscales: posture and gait disturbance, kinetic functions, speech disorders and oculomotor disorders. Subscale scores are summed to give a total score ranging from 0 to 100. High scores indicated worse ataxia. The Perdue Pegboard test, which assesses speed of performance, delicate movements and manipulative dexterity, was included in the course of the study. This test assesses the ability of the participant to insert as many nails as possible into preset holes, linearly dug in a wooden board in a limited space of time (20 seconds with the two hands, separately and together). The tests were administered by the same investigator. Patients were monitored for neutropenia, agranulocytosis, musculoskeletal pains and zinc deficiency and had weekly blood counts, plasma iron, serum ferritin and transferrin concentration measurements, as well as assessments of renal and liver function.

### MRI measurements

Multiple-gradient echo sequence was used to monitor the iron concentrations in the brain (Waldvogel et al. Increased iron in the dentate nucleus of patients with Friedrich's ataxia. Ann Neurol 1999;46:123-5), using a 1.5T Signa unit (GE Medical Systems, Milwaukee, WI, USA) using phase array head coil. Deep gray structures were localized by using a T2*-weighted echoplanar sequence (TR 3000 ms, TE 60 ms, eight averages, field of view : 24 cm, 256x224 matrix and slice thickness : 5mm, 24 slices/1.1 min). A voxel englobing the left and right nuclei was positioned on the largest section of dentate nuclei (dimensions 6 x 3 x 2 cm³). Data acquisition allowing iron monitoring was performed by using a single-slice multi-gradient-echo sequence (TR: 400 ms, flip angle: 50° to maximize gray matter signal, acquisition time: 3 minutes).

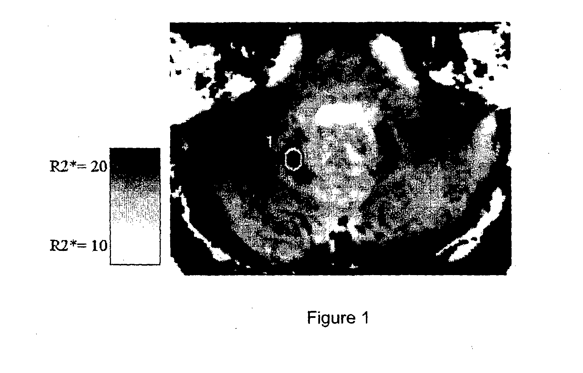

The determination of R2* was performed by using data of the multi-gradient echo sequence. In each pixel, the signals Si of the ten images obtained at echo times TEi (-i = 1.10) were used to calculate the parameter R2* by adjustment of the signal decay according to the equation Si = So.exp (-R2*.TEi). A parametric image of local R2* values was calculated with the same spatial resolution than the native images. The mean value of R2* was calculated in various regions of interest, all determined by the same experimental radiologist. For dentate nuclei, elliptic regions of 24mm² were laid at the center of each dentate nucleus visualized as a low-signal area (Figure 1). The position of the region of interest was selected in order to minimize R2* variance. Circular regions of 24mm² were drawn in the white matter of cerebellar hemispheres posterior to the dentate nuclei, a region where iron concentration is assumed to be low, and in pallidal and thalamic nuclei.

### Statistical analyses

At the basal state, R2* values in the different structures were compared using a generalised estimating equation (GEE) model, taking into account the individual levels with both the cerebral structure and the side as categorical covariates. For each cerebral structure, MRI repeated measurements over time were analyzed using a GEE model with the time of measurement as factor, while keeping a correlation structure between the values from the same individual. Individual contributions were weighted using the inverse of the variance of R2* in the corresponding region of interest. All calculations were carried out using the GEE procedure from the geepack library, R statistical package (http://www.R-project.org). A p value < 0.05 was considered significant. Tests were adjusted for multiple comparisons according to the Bonferroni rule.

### Results

Based on pharmacokinetic studies in thalassemia subjects, the first patient (P1) was included at an initial dose of 80 mg/kg/day of deferiprone. The rationale for using this dose was that CNS concentrations would be expected to be substantially lower than serum concentrations and that concentrations within the mitochondria would be expected to be even lower yet. Thus higher doses might be necessary to have the requisite iron chelating effect within the mitochondria. However, this dose, comparable to that used in patients with heavy iron loading, such as thalassemia, led to a series of undesirable events commencing on day eight, that finally led to termination of drug administration at day 17 in the first patient (P1). In addition, deferiprone administration to a second patient (P2) who had been treated for only 2 days at the same dose, was stopped. These adverse events, including: fatigue, headache, nausea, dizziness, then floppiness, poor head control, abnormal eye movements and fluctuating consciousness, slowly reversed on drug termination. These events have never been reported in patients with generalized iron overload, such as those with transfusion-dependent thalassemia receiving deferiprone, suggesting there may be a lower threshold level for toxicity of the drug in Friedreich ataxia, where no general iron overload is present. These results seemed to support the current thinking that iron chelators would not be beneficial in Friedreich ataxia because of the inability to separate mitochondrial from cytosolic chelation of iron, and because iron is a critical element of many biochemical processes required in intermediary metabolism (Richardson. Friedreich's ataxia: iron chelators that target the mitochondrion as a therapeutic strategy? Expert Opin Investig Drugs. 2003 Feb;12:235-45).

A re-evaluation of both the previously-generated data and the disposition kinetics of deferiprone was undertaken and a decision was made to resume the trial at a quarter of the initial dose (20 mg/kg/day) in new patients.

Within a few weeks, unexpected neurological improvement was noted by the parents and uninformed relatives in all treated patients. Constipation, incontinence and some subjective signs of peripheral neuropathy disappeared in 1-2 months, all without signs of toxicity, other than minor adverse effects, such as nausea. Limb extremities, which were cold, mottled and hypersensitive, warmed up with the reported sensation of feeling one's feet and floor surface again. Delicate movements and manipulative dexterity (e.g., handwriting, keyboard typing, hairdressing, eye make-up, etc.) and speech fluency were reportedly improved in several patients (Table -Attachment 1). General strength, quality of sitting, standing and facility of transfers (from bed to wheelchair or toilets) also improved. The youngest (14 years) and longest treated patients (P1-P3, 4-5 months) were also the ones who benefited most from the trial in terms of delicate movements, balance, stability and autonomy, suggesting that efficacy might be higher in younger patients. It is unlikely that these observations were due to a placebo effect as they were repeatedly and convergently reported by several uninformed parents (Table- Attachment 1). These features also yielded mild changes of the ICARS scores during the short period of the trial.

Based on the apparent lack oftoxicity at the low dose and evidence of possible clinical efficacy, two additional patients were included at a 50% higher dose of deferiprone (30 mg/kg/day) and the Perdue Pegboard test was included in order to assess the impact of the drug on manipulative dexterity. Results with this group confirmed the above observations in terms of tolerance and relative efficacy of deferiprone and showed an improvement of speed of performances and dexterity (number of nails inserted with the two hands alone + together, before and 1-2 months after onset of the trial, respectively (Table-Attachment 1).

MRI imaging of the brain iron-induced signal showed that R2* values in dentate nuclei were higher in Friedreich ataxia adolescents than in non-affected adolescents (R2*=17.4±1.6s⁻¹ and 13.7±0.6 s⁻¹ in patients and controls respectively, p<0.001). No correlation with age or brain side was noted and no significant variations of R2* in pallidal nuclei were observed (not shown). This observation supports the view that brain iron accumulation is an early event in Friedreich ataxia, contradicting the results initially reported in Friedreich ataxia knockout animals.

Deferiprone administration significantly decreased the relaxation rate R2*, after one month (16.6±1.3 s⁻¹), two months (15.9±0.6 s⁻¹) and four months of drug administration (Figure-2). Moreover, no short-term difference between the two doses of deferiprone tested was observed (20 and 30 mg/kg/day, Figure-2). No significant R2* changes were observed in pallidal nuclei, thalami and cerebellar white matter regions. Finally, the administration of the iron chelator had a negligible impact on the levels of hyposideremic anemia and hypoferritinemia, which remained essentially unchanged regardless of the dose of deferiprone (Table- Attachment 1).

The first drug treatment for some symptoms of Friedreich ataxia, although not yet approved by any regulatory body, is a quinone analogue (idebenone, Takeda) and acts as a potent free-radical scavenger, protecting heart muscle from iron-induced injury (Rustin et al. Effect of idebenone on cardiomyopathy in Friedreich's ataxia: a preliminary study. Lancet 1999;35:477-9). Long-term-idebenone administration improves cardiomyopathy, but has failed to improve or even stabilize the course of neurological symptoms. Increased survival, in the absence of significant improvement in the debilitating CNS sequelae of the disease, may not be a true benefit for the patients or their caregivers. A treatment is needed to ameliorate both the cardiovascular and CNS symptoms and the sum of the data provided above, together with the relevant findings of others demonstrate highly permeable, orally absorbed iron chelators, particularly deferiprone, are likely to have a powerful effect in ameliorating the symptoms of Friedreich ataxia.

Referring to Figure 1 there is shown an MRI visualisation of iron accumulation in dentate nuclei of patients with Friedreich ataxia. A parametric image of R2* values in posterior fossa, derived from the multi-gradient echo sequence done at 1.5 Tesla is shown. Dentate nuclei with high R2* values (measured in the elliptic region, here 17 s-1) appear as darker than the surrounding cerebellum. R2* values in the adjacent control region of interest 13 s-1, indicate good regional homogeneity of the magnetic field.

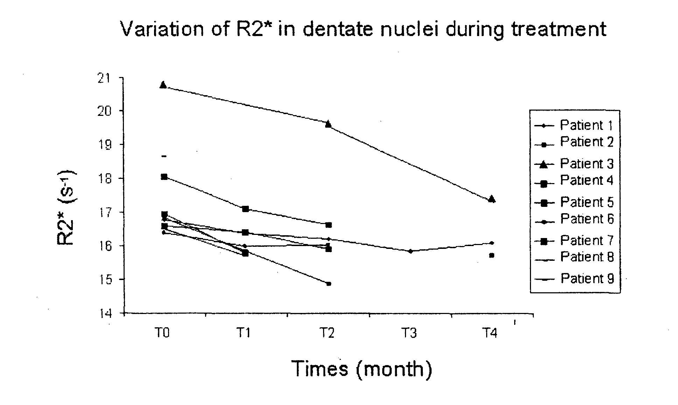

Referring to Figure 2 there is represented a Time course of mean R2* values in the left and right dentate nuclei of patients with Friedreich ataxia receiving deferiprone. The values of R2* in dentate nuclei reflect iron content before and after 1-5 months oral deferiprone administration (20-30mg/kg/day).

**Table 1: As per the discussion above the table below shows the age and sex of the patients, the age at onset of the disease, duration of ataxia, size of the GAA expansion in the frataxin gene, the doses and duration of deferiprone (DFP) administration, the ICARS score and biological parameters prior to and after the trial (bold characters). The observations of the parents are also given.**

| | DFP (mg/kg) Sex | Age (yrs) | Disease onset (yrs) | Ataxia duration (yrs) | GAA size (kb) | Posture and gait disturbance | | | | |
|---|---|---|---|---|---|---|---|---|---|---|
| | | | | | | walking | gait speed | spread feet | standing eyes open | 7 items (/34) |
| CS | 80 | 19 | 7 | 12 | 2.5/3.6 | | | | | |
| | 17 d | | | | | | | | | |
| | F | | | | | | | | | |
| DL | 80 | 18 | 5 | 13 | 2.7/3 | | | | | |
| | 2 d | | | | | | | | | |
| | M | | | | | | | | | |
| KH | 20 | 14 | 7 | 7 | 2/2.8 | | | | | |
| | 5 mth | | | | | | | | | |
| | M | | | | | | | | | |
| MT | 20 | 17 | 9 | 8 | 2.5/2.6 | | | | | |
| | 5 mth | | | | | 7 | 3 | 3 | 4 | 28 |
| | F | | | | | **7** | **3** | **2** | **4** | **27** |
| NG | 20 | 20 | 14 | 6 | 0.75/0.75 | | | | | |
| | 4 mth | | | | | 3 | 1 | 3 | 2 | 12 |
| | F | | | | | **2** | **1** | **3** | **1** | **9** |
| YL | 20 | 13 | 5 | 8 | 2.4/3.9 | | | | | |
| | 3 mth | | | | | 7 | 4 | 4 | 6 | 33 |
| | M | | | | | 7 | 4 | 4 | 6 | 32 |
| KS | 20 | 18 | 10 | 8 | 2.2/3.7 | | | | | |
| | 2 mth | | | | | 3 | 1 | 2 | 3 | 16 |
| | M | | | | | **3** | **1** | **2** | **1** | **13** |
| MV | 20 | 23 | 12 | 11 | 0.45/0.93 | | | | | |
| | 2 mth | | | | | 7 | 2 | 3 | 5 | 17 |
| | F | | | | | 7 | 2 | 3 | 4 | 16 |
| MC | 30 | 18 | 11 | 7 | 1.6/3.6 | | | | | |
| | 1 mth | | | | | 2 | 2 | 1 | 2 | 11 |
| | F | | | | | **2** | **2** | **0** | **1** | **8** |
| SB | 30 | 15 | 12 | 3 | G130V/????? | | | | | |
| | 1 mth | | | | | 7 | 1 | 2 | 3 | 20 |
| | F | | | | | 7 | **1** | **2** | **2** | **17** |

| | Kinetic function (/29) | Speech disorder (/8) | Oculomotor disorder (/6) | Total ICARS (/100) | Perdue pegboard | Hemoglobin (g/dl) Plasma iron (µmol/l) Ferritin (µg/l) | Observation |
|---|---|---|---|---|---|---|---|
| | | | | | | | |
| CS | | | | | | | undesirable event at d 17: fatigue, headache, nausea, diziness, flopiness, abnormal eye movement, fluctuating consciousness |
| DL | | | | | | | premature suspension at day 2 |
| KH | | | | | | 14, **13** | improved gait, writing, delicate movements, can move her toes, feels her feet, warming up of feet |
| | | | | | | 14, **9** | |
| | | | | | | 106, **13** | |
| MT | | | | | | 13, **11** | improved gait, writing, delicate movements, can move her toes, feels her feet, warming up of feet |
| | 8 | 1 | 2 | 39 | / | 7, **4** | |
| | 7 | **0** | **2** | **37** | | 8, **4** | |
| NG | | | | | | 14, **12** | no more incontinence, improved gait and balance, warming up of feet |
| | 7 | 2 | 2 | 23 | / | 27, **6** | |
| | 7 | **2** | **2** | **20** | | 15, **6** | |
| YL | | | | | | 13, **12** | no more fatigue, improved delicate movements |
| | 21 | 4 | 3 | 61 | / | 21, **14** | |
| | 21 | 4 | 2 | 59 | | 16, **8** | |
| KS | | | | | | 16, **15** | |
| | 13 | 2 | 0 | 31 | 37 | 16, **16** | |
| | **12** | **2** | **0** | **27** | **29** | 19, **15** | |
| MV | | | | | | 13, **13** | |
| | 11 | 3 | 0 | 31 | 37 | 26, **4** | |
| | 11 | 2 | 0 | 29 | 44 | **12, 7** | |
| MC | | | | | | 12, **10** | no more constipation, incontinence and bending, improved balance and gait |
| | 8 | 2 | 2 | 23 | | 16, **13** | |
| | **8** | **2** | **2** | **20** | | 5, **4** | |
| SB | | | | | | 13, **13** | improved gait and standing, no more constipation |
| | 9 | 0 | 0 | 29 | 52 | 18, **22** | |
| | **9** | **0** | **0** | **26** | **39** | 117, **114** | |

While the MRI results support the view that deferiprone has affected the intracellular levels of iron in the brain known to be altered in Friedreich ataxia, the clinical finding of changes in bodily function are the mainstay of this invention, showing true benefit in the well-being of these patients.

At the time of undertaking these studies, teaching in the field indicated that currently available iron chelators, such as deferiprone, would not be beneficial, as stated by Richardson "Indeed, standard chelation regimens will probably not work in FA, as these patients do not exhibit gross iron-loading." (Richardson. Friedreich's ataxia: iron chelators that target the mitochondrion as a therapeutic strategy? Expert Opin Investig Drugs. 2003;12:235-45.) This is remarkable in that 2 years earlier Richardson and others were convinced the iron chelation might work, and proposed a rationale why deferiprone might possibly be beneficial (Richardson et al. Development of potential iron chelators for the treatment of Friedreich's ataxia: ligands that mobilize mitochondrial iron. Biochimica et Biophysica Acta 2001;1536;133-140). By 2004, he had completely discounted deferiprone and did not even mention it in a review of potential chelators in Friedreich ataxia (Richardson DR. Novel chelators for central nervous system disorders that involve alterations in the metabolism of iron and other metal ions. Ann N Y Acad Sci. 2004;1012:326-41).

In light of the results obtained with respect to deferiprone in treating Friedreich Ataxia, and its capability of entering cells and removing iron from the mitochondria, it is clear that the other above-mentioned chelator, deferasirox, which is able to cross membranes and also can reduce intramitochondrial iron load (Glickstein et al. Intracellular labile iron pools as direct targets of iron chelators: a fluorescence study of chelator action in living cells. Blood 2005;106:3242-50), should achieve similar results.

Since the key factors in this discovery relate to the accumulation of iron in subcellular compartments of the brain, including the mitochondria, and the ability of cell penetrant oral iron chelators, like deferiprone and deferasirox, to remove iron from these compartments, it is reasonable to conclude that other conditions where mishandling of intracellular iron is a key factor in the development of the pathology, would also benefit from treatment with deferiprone or deferasirox. Based on MRI assessment of increased iron in various cells within the brain, in the absence of generalized iron overload, this discovery can be extended to the use of deferiprone or deferasirox in the following conditions: Friedreich Ataxia, Huntington's disease, Parkinson's disease, Alzheimer's disease, multiple sclerosis, hemochromatosis, Hallervorden-Spatz, Down syndrome, and in the eye for people with macular degeneration (Haacke et al. Imaging iron stores in the brain using magnetic resonance imaging. Magn Reson Imaging. 2005;23:1-25).

## Claims

1. Deferiprone, or a physiologically acceptable salt thereof, for use in the treatment of Friedreich ataxia resulting from mitochondrial iron-induced damage in the absence of generalised iron overload in a patient by daily administration in the range of 20 mg/kg to 30 mg/kg.

2. Deferiprone, or a physiologically acceptable salt thereof, according to claim 1 for use by daily administration of 20 mg/kg.

3. Deferiprone, or a physiologically acceptable salt thereof, according to claim 1 for use by daily administration of 30 mg/kg.

4. Deferiprone, or a physiologically acceptable salt thereof, according to any one of claims 1 to 3 for administration intravenously, transdermally, rectally, orally, bucally, or aurally.

5. Deferiprone, or a physiologically acceptable salt thereof, according to claim 4 for administration orally.

6. Deferiprone, or a physiologically acceptable salt thereof, according to claim 4 or 5 wherein the formulation comprises a modified release formulation, including sustained release.

7. Deferiprone, or a physiologically acceptable salt thereof, according to claim 4 or 5 for administration in addition to other regimens.

8. Deferiprone, or a physiologically acceptable salt thereof, according to claim 5 in an oral dosage form with other excipients.

## Patentansprüche

1. Deferipron oder ein physiologisch unbedenkliches Salz davon zur Verwendung bei der Behandlung von durch Eisen induzierte Schädigung der Mitochondrien verursachter Friedreich-Ataxie in Abwesenheit einer generalisierten Eisenüberlastung bei einem Patienten durch tägliche Verabreichung im Bereich von 20 mg/kg bis 30 mg/kg.

2. Deferipron oder ein physiologisch unbedenkliches Salz davon nach Anspruch 1 zur Verwendung durch tägliche Verabreichung von 20 mg/kg.

3. Deferipron oder ein physiologisch unbedenkliches Salz davon nach Anspruch 1 zur Verwendung durch tägliche Verabreichung von 30 mg/kg.

4. Deferipron oder ein physiologisch unbedenkliches Salz davon nach einem der Ansprüche 1 bis 3 zur intravenösen, transdermalen, rektalen, oralen, bukalen oder auralen Verabreichung.

5. Deferipron oder ein physiologisch unbedenkliches Salz davon nach Anspruch 4 zur oralen Verabreichung.

6. Deferipron oder ein physiologisch unbedenkliches Salz davon nach Anspruch 4 oder 5, wobei die Formulierung eine Formulierung mit modifizierter Freisetzung einschließlich anhaltender Freisetzung umfasst.

7. Deferipron oder ein physiologisch unbedenkliches Salz davon nach Anspruch 4 oder 5 zur Verabreichung zusätzlich zu anderen Verabreichungsprotokollen.

8. Deferipron oder ein physiologisch unbedenkliches Salz davon nach Anspruch 5 in oraler Verabreichungsform mit anderen Exzipienten.

## Revendications

1. Défériprone, ou un sel physiologiquement acceptable de celle-ci, pour utilisation dans le traitement de l'ataxie de Friedreich résultant de dommages mitochondriaux induits par le fer en l'absence d'une surcharge de fer généralisée chez un patient par administration quotidienne dans la plage de 20 mg/kg à 30 mg/kg.

2. Défériprone, ou un sel physiologiquement acceptable de celle-ci, selon la revendication 1 pour utilisation par administration quotidienne de 20 mg/kg.

3. Défériprone, ou un sel physiologiquement acceptable de celle-ci, selon la revendication 1 pour utilisation par administration quotidienne de 30 mg/kg.

4. Défériprone, ou un sel physiologiquement acceptable de celle-ci, selon l'une quelconque des revendications 1 à 3 pour administration par voie intraveineuse, transdermique, rectale, orale, buccale, ou auriculaire.

5. Défériprone, ou un sel physiologiquement acceptable de celle-ci, selon la revendication 4 pour administration par voie orale.

6. Défériprone, ou un sel physiologiquement acceptable de celle-ci, selon la revendication 4 ou 5, la formulation comprenant une formulation à libération modifiée, comprenant une libération prolongée.

7. Défériprone, ou un sel physiologiquement acceptable de celle-ci, selon la revendication 4 ou 5 pour administration en plus d'autres régimes.

8. Défériprone, ou un sel physiologiquement acceptable de celle-ci, selon la revendication 5 dans une forme pharmaceutique orale avec d'autres excipients.
